# EUROPEAN PATENT SPECIFICATION

(11) **EP 3 893 871 B1**
(45) Date of publication and mention of the grant of the patent: **10.04.2024**
(21) Application number: 19813747.3
(22) Date of filing: 27.11.2019
(51) Int. Cl.: A61K 31/422, C07D 413/14, A61P 29/00

(54) **BENZIMIDAZOLONE DERIVATIVES, AND ANALOGUES THEREOF, AS IL-17 MODULATORS**
BENZIMIDAZOLONDERIVATE UND ANALOGE DAVON ALS IL-17-MODULATOREN
DÉRIVÉS DE BENZIMIDAZOLONE, ET ANALOGUES DE CEUX-CI, EN TANT QUE MODULATEURS D'IL-17

(30) Priority: 11.12.2018 GB 201820165
(43) Date of publication of application: 20.10.2021
(73) Proprietor: UCB Biopharma SRL, 1070 Brussels (BE)
(72) Inventor: LECOMTE, Fabien Claude, Slough Berkshire SL1 3WE (GB); SELBY, Matthew Duncan, Slough Berkshire SL1 3WE (GB)
(74) Representative: UCB Intellectual Property
(86) International application number: PCT/EP2019/082774
(87) International publication number: WO 2020/120140

(56) References cited:
- US-A- 4 144 341

## Description

The present invention relates to heterocyclic compounds, and to their use in therapy. More particularly, this invention is concerned with pharmacologically active benzimidazole derivatives, and analogues thereof. These compounds act as modulators of IL-17 activity, and are accordingly of benefit as pharmaceutical agents for the treatment and/or prevention of pathological conditions, including adverse inflammatory and autoimmune disorders.

IL-17A (originally named CTLA-8 and also known as IL-17) is a pro-inflammatory cytokine and the founder member of the IL-17 family (Rouvier et al., J. Immunol., 1993, 150, 5445-5456). Subsequently, five additional members of the family (IL-17B to IL-17F) have been identified, including the most closely related, IL-17F (ML-1), which shares approximately 55% amino acid sequence homology with IL-17A (Moseley et al., Cytokine Growth Factor Rev., 2003, 14, 155-174). IL-17A and IL-17F are expressed by the recently defined autoimmune related subset of T helper cells, Th17, that also express IL-21 and IL-22 signature cytokines (Korn et al., Ann. Rev. Immunol., 2009, 27, 485-517). IL-17A and IL-17F are expressed as homodimers, but may also be expressed as the IL-17A/F heterodimer (Wright et al., J. Immunol., 2008, 181, 2799-2805). IL-17A and F signal through the receptors IL-17R, IL-17RC or an IL-17RA/RC receptor complex (Gaffen, Cytokine, 2008, 43, 402-407). Both IL-17A and IL-17F have been associated with a number of autoimmune diseases.

The compounds in accordance with the present invention, being potent modulators of human IL-17 activity, are therefore beneficial in the treatment and/or prevention of various human ailments, including inflammatory and autoimmune disorders.

Furthermore, the compounds in accordance with the present invention may be beneficial as pharmacological standards for use in the development of new biological tests and in the search for new pharmacological agents. Thus, the compounds of this invention may be useful as radioligands in assays for detecting pharmacologically active compounds.

WO 2013/116682 and WO 2014/066726 relate to separate classes of chemical compounds that are stated to modulate the activity of IL-17 and to be useful in the treatment of medical conditions, including inflammatory diseases.

Co-pending international patent application PCT/EP2018/065558 (published on 20 December 2018 as WO 2018/229079) describes spirocyclic oxoindoline derivatives, and analogues thereof, that are potent modulators of human IL-17 activity, and are therefore beneficial in the treatment of human ailments, including inflammatory and autoimmune disorders.

Co-pending international patent application PCT/EP2019/050594 (published on 18 July 2019 as WO 2019/138017) describes substituted fused bicyclic imidazole derivatives, including benzimidazole derivatives and analogues thereof, that are potent modulators of human IL-17 activity, and are therefore beneficial in the treatment of human ailments, including inflammatory and autoimmune disorders.

None of the prior art available to date, however, discloses or suggests the precise structural class of benzimidazole derivatives, and analogues thereof, as provided by the present invention.

The present invention provides a compound of formula (IA) or an *N-*oxide thereof, or a pharmaceutically acceptable salt thereof: 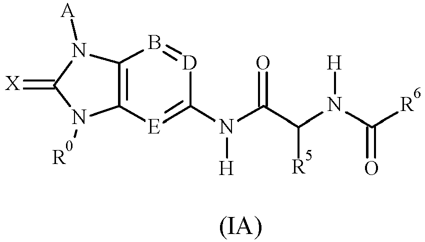 wherein
A represents oxetanyl or tetrahydropyranyl, either of which groups may be optionally substituted by one, two or three substituents independently selected from C₁₋₆ alkyl, oxo and imino;
B represents C-R²;
D represents C-R³;
E represents C-R⁴;
X represents O or S;
R⁰ represents hydrogen or Cₗ-₆ alkyl;
R² represents hydrogen or halogen;
R³ represents hydrogen or halogen;
R⁴ represents hydrogen;
R⁵ represents C₃₋₉ cycloalkyl, which group may be optionally substituted by one, two or three substituents independently selected from halogen, cyano, C₁₋₆ alkyl, trifluoromethyl, phenyl, hydroxy, C₁₋₆ alkoxy and aminocarbonyl; and
R⁶ represents heteroaryl, which group may be optionally substituted by one, two or three substituents independently selected from C₁₋₆ alkyl.

The present invention also provides a compound of formula (IA) as defined above or an N-oxide thereof, or a pharmaceutically acceptable salt thereof, for use in therapy.

The present invention also provides a compound of formula (IA) as defined above or an N-oxide thereof, or a pharmaceutically acceptable salt thereof, for use in the treatment and/or prevention of inflammatory or autoimmune disorders.

For use in medicine, the salts of the compounds of formula (IA) will be pharmaceutically acceptable salts. Other salts may, however, be useful in the preparation of the compounds of formula (IA) or of their pharmaceutically acceptable salts. Standard principles underlying the selection and preparation of pharmaceutically acceptable salts are described, for example, in Handbook of Pharmaceutical Salts: Properties, Selection and Use, ed. P.H. Stahl & C.G. Wermuth, Wiley-VCH, 2002. Suitable pharmaceutically acceptable salts of the compounds of formula (IA) include acid addition salts which may, for example, be formed by mixing a solution of a compound of formula (IA) with a solution of a pharmaceutically acceptable acid.

There may also be contemplated co-crystals of the compounds of formula (IA) above. The technical term "co-crystal" is used to describe the situation where neutral molecular components are present within a crystalline compound in a definite stoichiometric ratio. The preparation of pharmaceutical co-crystals enables modifications to be made to the crystalline form of an active pharmaceutical ingredient, which in turn can alter its physicochemical properties without compromising its intended biological activity (see Pharmaceutical Salts and Co-crystals, ed. J. Wouters & L. Quere, RSC Publishing, 2012).

Suitable alkyl groups which may be present on the compounds of use in the invention include straight-chained and branched Cₗ-₆ alkyl groups, for example C₁₋₄ alkyl groups. Typical examples include methyl and ethyl groups, and straight-chained or branched propyl, butyl and pentyl groups. Particular alkyl groups include methyl, ethyl, n-propyl, isopropyl, *n*-butyl, *sec*-butyl, isobutyl, *tert*-butyl, 2,2-dimethylpropyl and 3-methylbutyl. Derived expressions such as "C₁₋₆ alkoxy", "C₁₋₆ alkylthio", "C₁₋₆ alkylsulphonyl" and "C₁₋₆ alkylamino" are to be construed accordingly.

The term "C₃₋₉ cycloalkyl" as used herein refers to monovalent groups of 3 to 9 carbon atoms derived from a saturated monocyclic hydrocarbon, and may comprise benzo-fused analogues thereof. Suitable C₃₋₉ cycloalkyl groups include cyclopropyl, cyclobutyl, benzocyclobutenyl, cyclopentyl, indanyl, cyclohexyl, tetrahydronaphthalenyl, cycloheptyl, benzocycloheptenyl, cyclooctyl and cyclononanyl.

The term "heteroaryl" as used herein refers to monovalent aromatic groups containing at least 5 atoms derived from a single ring or multiple condensed rings, wherein one or more carbon atoms have been replaced by one or more heteroatoms selected from oxygen, sulphur and nitrogen. Suitable heteroaryl groups include furyl, benzofuryl, dibenzofuryl, thienyl, benzothienyl, thieno[2,3-*c*]pyrazolyl, thieno[3,4-*b*][1,4]dioxinyl, dibenzothienyl, pyrrolyl, indolyl, pyrrolo[2,3-*b*]pyridinyl, pyrrolo[3,2-*c*]pyridinyl, pyrrolo[3,4-*b*]pyridinyl, pyrazolyl, pyrazolo[1,5-*a*]pyridinyl, pyrazolo[3,4-*d*]pyrimidinyl, pyrazolo[1,5-a]pyrazinyl, indazolyl, 4,5,6,7-tetrahydroindazolyl, oxazolyl, benzoxazolyl, isoxazolyl, thiazolyl, benzothiazolyl, isothiazolyl, imidazolyl, benzimidazolyl, imidazo-[2,1-*b*]thiazolyl, imidazo[1,2-*a*]pyridinyl, imidazo[4,5-*b*]pyridinyl, imidazo[1,2-*b*]-pyridazinyl, purinyl, imidazo[1,2-*a*]pyrimidinyl, imidazo[1,2-*a*]pyrazinyl, oxadiazolyl, thiadiazolyl, triazolyl, [1,2,4]triazolo[1,5-*a*]pyrimidinyl, benzotriazolyl, tetrazolyl, pyridinyl, quinolinyl, isoquinolinyl, naphthyridinyl, pyridazinyl, cinnolinyl, phthalazinyl, pyrimidinyl, quinazolinyl, pyrazinyl, quinoxalinyl, pteridinyl, triazinyl and chromenyl groups.

The term "halogen" as used herein is intended to include fluorine, chlorine, bromine and iodine atoms, typically fluorine, chlorine or bromine.

Where the compounds of formula (IA) have one or more asymmetric centres, they may accordingly exist as enantiomers. Where the compounds in accordance with the invention possess two or more asymmetric centres, they may additionally exist as diastereomers. The invention is to be understood to extend to the use of all such enantiomers and diastereomers, and to mixtures thereof in any proportion, including racemates. Formula (IA) and the formulae depicted hereinafter are intended to represent all individual stereoisomers and all possible mixtures thereof, unless stated or shown otherwise. In addition, compounds of formula (IA) may exist as tautomers, for example keto (CH₂C=O)↔enol (CH=CHOH) tautomers or amide (NHC=O)↔hydroxyimine (N=COH) tautomers. Formula (IA) and the formulae depicted hereinafter are intended to represent all individual tautomers and all possible mixtures thereof, unless stated or shown otherwise.

It is to be understood that each individual atom present in formula (IA), or in the formulae depicted hereinafter, may in fact be present in the form of any of its naturally occurring isotopes, with the most abundant isotope(s) being preferred. Thus, by way of example, each individual hydrogen atom present in formula (IA), or in the formulae depicted hereinafter, may be present as a ¹H, ²H (deuterium) or ³H (tritium) atom, preferably ¹H. Similarly, by way of example, each individual carbon atom present in formula (IA), or in the formulae depicted hereinafter, may be present as a ¹²C, ¹³C or ¹⁴C atom, preferably ¹²C.

Suitable examples of particular substituents on integer A include one, two or three substituents independently selected from methyl, oxo and imino.

Typical values of integer A include oxetanyl, tetrahydropyranyl and (methyl)tetrahydropyranyl.

Particular values of integer A include oxetanyl and tetrahydropyranyl.

In a first embodiment, X represents O. In a second embodiment, X represents S.

In a first embodiment, R⁰ represents hydrogen. In a second embodiment, R⁰ represents C₁₋₆ alkyl, especially methyl.

Suitably, R⁰ represents hydrogen or methyl.

In a first embodiment, R² represents hydrogen. In a second embodiment, R² represents halogen. In a first aspect of that embodiment, R² represents fluoro. In a second aspect of that embodiment, R² represents chloro.

Suitably, R² represents hydrogen or fluoro.

In a first embodiment, R³ represents hydrogen. In a second embodiment, R³ represents halogen. In a first aspect of that embodiment, R³ represents fluoro. In a second aspect of that embodiment, R³ represents chloro.

Appositely, R³ represents hydrogen, fluoro or chloro.

Suitably, R³ represents hydrogen or fluoro.

Typical values of R⁵ include cyclobutyl, cyclopentyl, cyclohexyl, cycloheptyl, cyclooctyl and cyclononanyl, any of which groups may be optionally substituted by one, two or three substituents as defined above.

Suitable values of R⁵ include cyclohexyl and cyclooctyl, either of which groups may be optionally substituted by one, two or three substituents as defined above.

Apposite values of R⁵ include cyclooctyl, which group may be optionally substituted by one, two or three substituents as defined above.

Suitable examples of specific substituents on R⁵ include one, two or three substituents independently selected from fluoro, chloro, bromo, cyano, methyl, trifluoromethyl, phenyl, hydroxy, methoxy, isopropoxy. *tert*-butoxy and aminocarbonyl, especially methyl.

Apposite values of R⁵ include *tert*-butoxymethylcyclobutyl, methylcyclobutyl, dimethylcyclobutyl, phenylcyclobutyl, cyclopentyl, methylcyclopentyl, cyclohexyl, difluorocyclohexyl, methylcyclohexyl, dimethylcyclohexyl, trifluoromethylcyclohexyl, cycloheptyl, cyclooctyl and cyclononanyl.

Favoured values of R⁵ include 4-methylcyclohexyl and cyclooctyl. In a first embodiment, R⁵ represents 4-methylcyclohexyl. In a second embodiment, R⁵ represents cyclooctyl.

Typical values of R⁶ include pyrrolyl, pyrazolyl, pyrazolo[1,5-a]pyridinyl, oxazolyl, isoxazolyl, thiazolyl, isothiazolyl, imidazolyl, oxadiazolyl, thiadiazolyl, triazolyl, tetrazolyl, pyridinyl, pyridazinyl and pyrimidinyl, any of which groups may be optionally substituted by one, two or three substituents as defined above.

Apposite values of R⁶ include pyrazolyl and isoxazolyl, either of which groups may be optionally substituted by one, two or three substituents as defined above.

Suitable values of R⁶ include isoxazolyl, which group may be optionally substituted by one, two or three substituents as defined above.

Typical examples of specific substituents on R⁶ include one, two or three substituents independently selected from methyl, ethyl, *n*-propyl, isopropyl, 2-methylpropyl, butan-2-yl and *tert*-butyl.

Suitable examples of specific substituents on R⁶ include one, two or three substituents independently selected from methyl and ethyl, especially methyl.

Illustrative values of R⁶ include methylpyrrolyl, methylpyrazolyl, dimethyl-pyrazolyl, ethylpyrazolyl, (ethyl)(fluoro)pyrazolyl, (ethyl)(methyl)pyrazolyl, *n-*propylpyrazolyl, isopropylpyrazolyl, 2-methylpropylpyrazolyl, butan-2-ylpyrazolyl, difluoromethylpyrazolyl, (difluoromethyl)(methyl)pyrazolyl, difluoroethylpyrazolyl, trifluoroethylpyrazolyl, trifluoropropylpyrazolyl, cyclopropylpyrazolyl, cyclobutyl-pyrazolyl, cyclopropylmethylpyrazolyl, hydroxyethylpyrazolyl, methoxyethylpyrazolyl, dimethylaminoethylpyrazolyl, tetrahydropyranylpyrazolyl, (methyl)(tetrahydropyranyl)-pyrazolyl, pyrazolo[1,5-*a*]pyridinyl, oxazolyl, methyloxazolyl, ethyloxazolyl, isoxazolyl, methylisoxazolyl, dimethylisoxazolyl, ethylisoxazolyl, isopropylisoxazolyl, *tert-*butyl-isoxazolyl, trifluoromethylisoxazolyl, cyclopropylisoxazolyl, cyclobutylisoxazolyl, methoxymethylisoxazolyl, aminomethylisoxazolyl, aminoisopropylisoxazolyl, thiazolyl, methylthiazolyl, dimethylthiazolyl, isothiazolyl, methylisothiazolyl, methylimidazolyl, methyloxadiazolyl, ethyloxadiazolyl, methylthiadiazolyl, methyltriazolyl, dimethyl-triazolyl, ethyltriazolyl, methyltetrazolyl, pyridinyl, methylpyridinyl, pyridazinyl, pyrimidinyl and methylpyrimidinyl.

Apposite values of R⁶ include methylpyrazolyl, ethylpyrazolyl, methylisoxazolyl and ethylisoxazolyl.

Representative values of R⁶ include methylisoxazolyl.

One sub-class of the compounds of formula (IA) above is represented by the compounds of formula (IIA), and pharmaceutically acceptable salts thereof: 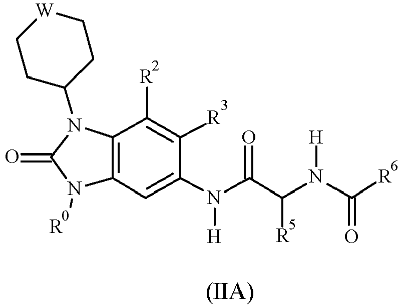 wherein
W represents O; and
R⁰, R², R³, R⁵ and R⁶ are as defined above.

Another sub-class of the compounds of formula (IA) above is represented by the compounds of formula (IIB), and pharmaceutically acceptable salts thereof: 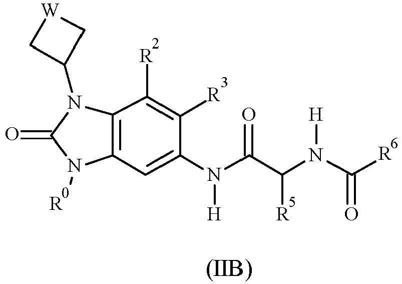 wherein W, R⁰, R², R³, R⁵ and R⁶ are as defined above.

Specific novel compounds in accordance with the present invention include each of the compounds whose preparation is described in the accompanying Examples, and pharmaceutically acceptable salts thereof.

The compounds in accordance with the present invention are beneficial in the treatment and/or prevention of various human ailments, including inflammatory and autoimmune disorders.

The compounds according to the present invention are useful in the treatment and/or prophylaxis of a pathological disorder that is mediated by a pro-inflammatory IL-17 cytokine or is associated with an increased level of a pro-inflammatory IL-17 cytokine. Generally, the pathological condition is selected from the group consisting of infections (viral, bacterial, fungal and parasitic), endotoxic shock associated with infection, arthritis, rheumatoid arthritis, psoriatic arthritis, systemic onset juvenile idiopathic arthritis (JIA), systemic lupus erythematosus (SLE), asthma, chronic obstructive airways disease (COAD), chronic obstructive pulmonary disease (COPD), acute lung injury, pelvic inflammatory disease, Alzheimer's Disease, Crohn's disease, inflammatory bowel disease, irritable bowel syndrome, ulcerative colitis, Castleman's disease, ankylosing spondylitis and other spondyloarthropathies, dermatomyositis, myocarditis, uveitis, exophthalmos, autoimmune thyroiditis, Peyronie's Disease, coeliac disease, gall bladder disease, Pilonidal disease, peritonitis, psoriasis, atopic dermatitis, vasculitis, surgical adhesions, stroke, autoimmune diabetes, Type I Diabetes, lyme arthritis, meningoencephalitis, immune mediated inflammatory disorders of the central and peripheral nervous system such as multiple sclerosis and Guillain-Barr syndrome, other autoimmune disorders, pancreatitis, trauma (surgery), graft-versus-host disease, transplant rejection, fibrosing disorders including pulmonary fibrosis, liver fibrosis, renal fibrosis, scleroderma or systemic sclerosis, cancer (both solid tumours such as melanomas, hepatoblastomas, sarcomas, squamous cell carcinomas, transitional cell cancers, ovarian cancers and hematologic malignancies and in particular acute myelogenous leukaemia, chronic myelogenous leukemia, chronic lymphatic leukemia, gastric cancer and colon cancer), heart disease including ischaemic diseases such as myocardial infarction as well as atherosclerosis, intravascular coagulation, bone resorption, osteoporosis, periodontitis, hypochlorhydia and pain (particularly pain associated with inflammation).

WO 2009/089036 reveals that modulators of IL-17 activity may be administered to inhibit or reduce the severity of ocular inflammatory disorders, in particular ocular surface inflammatory disorders including Dry Eye Syndrome (DES). Consequently, the compounds in accordance with the present invention are useful in the treatment and/or prevention of an IL-17-mediated ocular inflammatory disorder, in particular an IL-17-mediated ocular surface inflammatory disorder including Dry Eye Syndrome. Ocular surface inflammatory disorders include Dry Eye Syndrome, penetrating keratoplasty, corneal transplantation, lamellar or partial thickness transplantation, selective endothelial transplantation, corneal neovascularization, keratoprosthesis surgery, corneal ocular surface inflammatory conditions, conjunctival scarring disorders, ocular autoimmune conditions, Pemphigoid syndrome, Stevens-Johnson syndrome, ocular allergy, severe allergic (atopic) eye disease, conjunctivitis and microbial keratitis. Particular categories of Dry Eye Syndrome include keratoconjunctivitis sicca (KCS), Sjögren syndrome, Sjögren syndrome-associated keratoconjunctivitis sicca, non-Sjögren syndrome-associated keratoconjunctivitis sicca, keratitis sicca, sicca syndrome, xerophthalmia, tear film disorder, decreased tear production, aqueous tear deficiency (ATD), meibomian gland dysfunction and evaporative loss.

Illustratively, the compounds of the present invention may be useful in the treatment and/or prophylaxis of a pathological disorder selected from the group consisting of arthritis, rheumatoid arthritis, psoriasis, psoriatic arthritis, systemic onset juvenile idiopathic arthritis (JIA), systemic lupus erythematosus (SLE), asthma, chronic obstructive airway disease, chronic obstructive pulmonary disease, atopic dermatitis, scleroderma, systemic sclerosis, lung fibrosis, inflammatory bowel diseases (including Crohn's disease and ulcerative colitis), ankylosing spondylitis and other spondyloarthropathies, cancer and pain (particularly pain associated with inflammation).

Suitably, the compounds of the present invention are useful in the treatment and/or prophylaxis of psoriasis, psoriatic arthritis or ankylosing spondylitis.

The present invention also provides a pharmaceutical composition which comprises a compound in accordance with the invention as described above, or a pharmaceutically acceptable salt thereof, in association with one or more pharmaceutically acceptable carriers.

Pharmaceutical compositions according to the invention may take a form suitable for oral, buccal, parenteral, nasal, topical, ophthalmic or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methyl cellulose); fillers (e.g. lactose, microcrystalline cellulose or calcium hydrogenphosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles or preservatives. The preparations may also contain buffer salts, flavouring agents, colouring agents or sweetening agents, as appropriate.

Preparations for oral administration may be suitably formulated to give controlled release of the active compound.

For buccal administration, the compositions may take the form of tablets or lozenges formulated in conventional manner.

The compounds according to the present invention may be formulated for parenteral administration by injection, e.g. by bolus injection or infusion. Formulations for injection may be presented in unit dosage form, e.g. in glass ampoules or multi-dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the compounds according to the present invention may also be formulated as a depot preparation. Such long-acting formulations may be administered by implantation or by intramuscular injection.

For nasal administration or administration by inhalation, the compounds according to the present invention may be conveniently delivered in the form of an aerosol spray presentation for pressurised packs or a nebuliser, with the use of a suitable propellant, e.g. dichlorodifluoromethane, fluorotrichloromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

For topical administration the compounds according to the present invention may be conveniently formulated in a suitable ointment containing the active component suspended or dissolved in one or more pharmaceutically acceptable carriers. Particular carriers include, for example, mineral oil, liquid petroleum, propylene glycol, polyoxyethylene, polyoxypropylene, emulsifying wax and water. Alternatively, the compounds according to the present invention may be formulated in a suitable lotion containing the active component suspended or dissolved in one or more pharmaceutically acceptable carriers. Particular carriers include, for example, mineral oil, sorbitan monostearate, polysorbate 60, cetyl esters wax, cetearyl alcohol, benzyl alcohol, 2-octyldodecanol and water.

For ophthalmic administration the compounds according to the present invention may be conveniently formulated as micronized suspensions in isotonic, pH-adjusted sterile saline, either with or without a preservative such as a bactericidal or fungicidal agent, for example phenylmercuric nitrate, benzylalkonium chloride or chlorhexidine acetate. Alternatively, for ophthalmic administration the compounds according to the present invention may be formulated in an ointment such as petrolatum.

For rectal administration the compounds according to the present invention may be conveniently formulated as suppositories. These can be prepared by mixing the active component with a suitable non-irritating excipient which is solid at room temperature but liquid at rectal temperature and so will melt in the rectum to release the active component. Such materials include, for example, cocoa butter, beeswax and polyethylene glycols.

The quantity of a compound according to the present invention required for the prophylaxis or treatment of a particular condition will vary depending on the compound chosen and the condition of the patient to be treated. In general, however, daily dosages may range from around 10 ng/kg to 1000 mg/kg, typically from 100 ng/kg to 100 mg/kg, e.g. around 0.01 mg/kg to 40 mg/kg body weight, for oral or buccal administration, from around 10 ng/kg to 50 mg/kg body weight for parenteral administration, and from around 0.05 mg to around 1000 mg, e.g. from around 0.5 mg to around 1000 mg, for nasal administration or administration by inhalation or insufflation.

If desired, a compound in accordance with the present invention may be co-administered with another pharmaceutically active agent, e.g. an anti-inflammatory molecule.

The compounds of formula (IA) above may be prepared by a process which comprises reacting a carboxylic acid of formula R^{a}CO₂H, or a salt thereof, e.g. a lithium salt thereof, with a compound of formula (III): 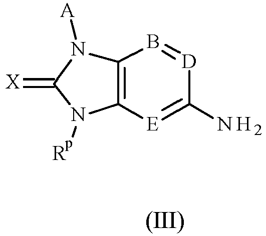 wherein A, B, D, E and X are as defined above, R^{a} represents -CH(R⁵)N(H)C(O)R⁶ in which R⁵ and R⁶ are as defined above, and R^{p} corresponds to the group R⁰ as defined above, or R^{p} represents a *N*-protecting group; followed, as necessary, by removal of the *N-*protecting group R^{p}.

The *N*-protecting group R^{p} will suitably be *tert*-butoxycarbonyl (BOC), benzyl, or 2-(trimethylsilyl)ethoxymethyl (SEM).

The reaction is conveniently accomplished in the presence of a coupling agent. Suitable coupling agents may comprise the following:
- 2-(7-aza-1*H*-benzotriazol-1-yl)-1,1,3,3-tetramethyluronium hexafluorophosphate (HATU);
- 2,4,6-tripropyl-1,3,5,2,4,6-trioxatriphosphorinane 2,4,6-trioxide (propylphosphonic anhydride); or
- a mixture of *N*-(3-dimethylaminopropyl)-*N'*-ethylcarbodiimide hydrochloride and 1-hydroxybenzotriazole.

Where compound (III) is reacted with a carboxylic acid of formula R^{a}CO₂H, the reaction is generally carried out in the presence of a base. Suitable bases include organic amines, e.g. a trialkylamine such as *N,N*-diisopropylethylamine or triethylamine. The reaction is conveniently performed at ambient temperature in a suitable solvent, e.g. a cyclic ether such as tetrahydrofuran, or a dipolar aprotic solvent such as *N,N*-dimethylformamide, or a chlorinated solvent such as dichloromethane.

Where compound (III) is reacted with the lithium salt of a carboxylic acid of formula R^{a}CO₂H, the reaction is generally carried out at ambient temperature in a suitable solvent, e.g. a dipolar aprotic solvent such as *N,N*-dimethylformamide, or a chlorinated solvent such as dichloromethane.

Where the *N*-protecting group R^{p} is BOC, the subsequent removal thereof may conveniently be effected by treatment with an acid, e.g. a mineral acid such as hydrochloric acid, or an organic acid such as trifluoroacetic acid.

Where the *N*-protecting group R^{p} is benzyl, the subsequent removal thereof may conveniently be effected by catalytic hydrogenation, typically by treatment with gaseous hydrogen in the presence of a hydrogenation catalyst, e.g. palladium on charcoal.

Where the *N*-protecting group R^{p} is SEM, the subsequent removal thereof may conveniently be effected by treatment with a fluoride salt, e.g. tetra-*n*-butylammonium fluoride; or by treatment with an acid, e.g. a mineral acid such as hydrochloric acid, or an organic acid such as trifluoroacetic acid.

The intermediates of formula R^{a}CO₂H where R^{a} is as defined above may be prepared by a two-step procedure which comprises: (i) reacting a carboxylic acid of formula R⁶-CO₂H with a compound of formula (IV): 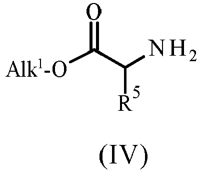 wherein Alk¹ represents C₁₋₄ alkyl, e.g. methyl, and R⁵ and R⁶ are as defined above; under conditions analogous to those described above for the reaction between compound (III) and a carboxylic acid of formula R^{a}CO₂H; and (ii) saponification of the resulting material by treatment with a base.

The saponification reaction in step (ii) will generally be effected by treatment with a base. Suitable bases include inorganic hydroxides, e.g. an alkali metal hydroxide such as lithium hydroxide. Where lithium hydroxide is employed in step (ii) of the above procedure, the product may be the lithium salt of the carboxylic acid of formula R^{a}CO₂H.

Step (ii) is conveniently effected at ambient temperature in water and a suitable organic solvent, e.g. a cyclic ether such as tetrahydrofuran, optionally in admixture with a C₁₋₄ alkanol such as methanol.

In another procedure, the compounds of formula (IA) above may be prepared by a process which comprises reacting an amide of formula R^{a}CONH₂ with a compound of formula (V): 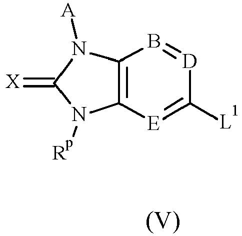 wherein A, B, D, E, X, R^{a} and R^{p} are as defined above, and L¹ represents a suitable leaving group; in the presence of a transition metal catalyst; followed, as necessary, by removal of the N-protecting group R^{p}.

The leaving group L¹ is suitably a halogen atom, e.g. chloro or bromo.

The transition metal catalyst is suitably [(2-di-*tert*-butylphosphino-3,6-dimethoxy-2',4',6'-triisopropyl-1,1'-biphenyl)-2-(2'-amino-1,1'-biphenyl)]palladium(II) methanesulfonate (tBuBrettPhos Pd G3), in which case the reaction will generally be performed in the presence of 2-(di-*tert*-butylphosphino)-2',4',6'- triisopropyl-3,6-dimethoxy-1,1'-biphenyl (tBuBrettPhos). The reaction is conveniently carried out at an elevated temperature in the presence of a base, e.g. an inorganic base such as potassium carbonate, in a suitable solvent, e.g. a lower alkanol such as *tert*-butanol.

Alternatively, the transition metal catalyst may suitably be tris(dibenzylideneacetone)dipalladium(0), in which case the reaction will generally be performed in the presence of 2-dicyclohexylphosphino-2',4',6'-triisopropylbiphenyl (XPhos) or 4,5-bis(diphenylphosphino)-9,9-dimethylxanthene (Xantphos). The reaction is conveniently carried out at an elevated temperature in the presence of a base, e.g. a carbonate salt such as potassium carbonate or cesium carbonate, in a suitable solvent, e.g. a cyclic ether such as 1,4-dioxane, or a C₁₋₆ alkanol such as *tert*-butanol.

The intermediates of formula (III) above may be prepared by reacting the corresponding compound of formula (V) above with sodium azide. The reaction will generally be accomplished in the presence of copper(I) iodide, L-proline and a base, e.g. an inorganic base such as potassium carbonate. The reaction is conveniently performed at an elevated temperature in a suitable solvent, e.g. an organic sulfoxide such as dimethyl sulfoxide.

The intermediates of formula (V) above wherein X is O may be prepared by reacting a compound of formula (VI): 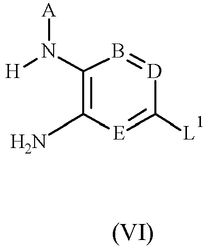 wherein A, B, D, E and L¹ are as defined above; with 1,1'-carbonyldiimidazole; followed, as necessary, by attachment of the *N*-protecting group R^{p}.

The reaction will conveniently be carried out at ambient temperature in a suitable solvent, e.g. a cyclic ether such as tetrahydrofuran.

Where the *N-*protecting group R^{p} is SEM, attachment thereof will suitably be effected by treatment with 2-(trimethylsilyl)ethoxymethyl chloride. The reaction is generally performed in the presence of a base, e.g. an inorganic base such as sodium hydride. The reaction will conveniently be carried out at ambient temperature in a suitable solvent, e.g. a dipolar aprotic solvent such as *N,N*-dimethylformamide.

The intermediates of formula (VI) above may be prepared by a two-step procedure from a compound of formula (VII): 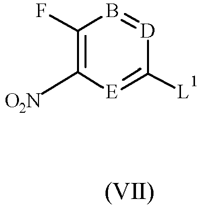 wherein B, D, E and L¹ are as defined above; which procedure comprises the following steps:
(i) reaction of compound (VII) with a compound of formula A-NH₂; and
(ii) treatment of the material thereby obtained with a reducing agent.

Step (i) is typically effected in the presence of a base, e.g. an inorganic base such as potassium carbonate. The reaction is conveniently carried out at an elevated temperature in a suitable solvent, e.g. a cyclic ether such as tetrahydrofuran.

The reducing agent of use in step (ii) suitably comprises a mixture of metallic zinc and ammonium formate. The reaction is conveniently carried out at ambient temperature in a suitable solvent, e.g. a lower alkanol such as methanol.

In another procedure, the compounds of formula (IA) above may be prepared by a process which comprises reacting a compound of formula (III) as defined above with a compound of formula (VIII): 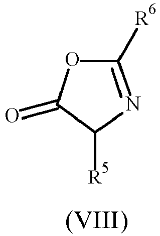 wherein R⁵ and R⁶ are as defined above; followed, as necessary, by removal of the *N-*protecting group R^{p}.

The reaction between compounds (III) and (VIII) will generally be performed in the presence of acetic acid. The reaction is conveniently carried out at an elevated temperature in a suitable solvent, e.g. a cyclic ether such as tetrahydrofuran.

In another procedure, the compounds of formula (IA) above may be prepared by a process which comprises reacting a carboxylic acid of formula R⁶-CO₂H with a compound of formula (X): 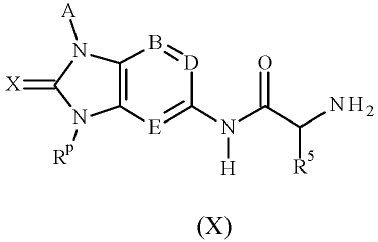 wherein A, B, D, E, X, R^{p}, R⁵ and R⁶ are as defined above; under conditions analogous to those described above for the reaction between compound (III) and a carboxylic acid of formula R^{a}CO₂H; followed, as necessary, by removal of the *N*-protecting group R^{p}.

The intermediates of formula (X) above may be prepared by reacting a compound of formula (III) as defined above with a compound of formula (XI), or a salt thereof, e.g. a lithium salt thereof: 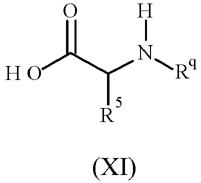 wherein R⁵ is as defined above, and R^{q} represents hydrogen or an *N*-protecting group; under conditions analogous to those described above for the reaction between compound (III) and a carboxylic acid of formula R^{a}CO₂H; followed, as necessary, by removal of the N-protecting group R^{q}.

The *N*-protecting group R^{q} will suitably be *tert*-butoxycarbonyl (BOC).

Where the *N*-protecting group R^{q} is BOC, the subsequent removal thereof may conveniently be effected by treatment with an acid, e.g. a mineral acid such as hydrochloric acid, or an organic acid such as trifluoroacetic acid.

Where they are not commercially available, the starting materials of formula (IV), (VII) and (XI) may be prepared by methods analogous to those described in the accompanying Examples, or by standard methods well known from the art.

It will be understood that any compound of formula (IA) initially obtained from any of the above processes may, where appropriate, subsequently be elaborated into a further compound of formula (IA) by techniques known from the art. By way of example, a compound comprising a N-BOC moiety (wherein BOC is an abbreviation for *tert-*butoxycarbonyl) may be converted into the corresponding compound comprising a N-H moiety by treatment with an acid, e.g. a mineral acid such as hydrochloric acid, or an organic acid such as trifluoroacetic acid.

A compound of formula (IA) wherein X is O may be converted into the corresponding compound wherein X is S by treatment with 2,4-bis(4-methoxyphenyl)-1,3-dithia-2,4-diphosphetane 2,4-disulfide (Lawesson reagent).

A compound comprising an amino (-NH₂) moiety may be acylated, e.g. acetylated, by treatment with a suitable acyl halide, e.g. acetyl chloride, typically in the presence of a base, e.g. an organic base such as *N,N*-diisopropylethylamine.

A compound which contains an N-H moiety may be alkylated, e.g. methylated, by treatment with the appropriate alkyl halide, e.g. iodomethane, typically at ambient temperature in the presence of a base, e.g. sodium hydride, in a suitable solvent, e.g. a dipolar aprotic solvent such as *N,N*-dimethylformamide.

A compound of formula (IA) wherein R² or R³ is hydrogen may be converted into the corresponding compound wherein R² or R³ is fluoro by treatment with Selectfluor^{™}.

A compound of formula (IA) wherein R² or R³ is hydrogen may be converted into the corresponding compound wherein R² or R³ is chloro by treatment with N-chlorosuccinimide, typically in the presence of acetic acid.

A compound containing the moiety -S- may be converted into the corresponding compound containing the moiety -S(O)- by treatment with 3-chloroperoxybenzoic acid. Likewise, a compound containing the moiety -S- or -S(O)- may be converted into the corresponding compound containing the moiety -S(O)₂- by treatment with 3-chloroperoxybenzoic acid.

A compound containing the moiety -S- may be converted into the corresponding compound containing the moiety -S(O)(NH)- by treatment with ammonium carbamate and (diacetoxyiodo)benzene.

Where a mixture of products is obtained from any of the processes described above for the preparation of compounds according to the invention, the desired product can be separated therefrom at an appropriate stage by conventional methods such as preparative HPLC; or column chromatography utilising, for example, silica and/or alumina in conjunction with an appropriate solvent system.

Where the above-described processes for the preparation of the compounds according to the invention give rise to mixtures of stereoisomers, these isomers may be separated by conventional techniques. In particular, where it is desired to obtain a particular enantiomer of a compound of formula (IA) this may be produced from a corresponding mixture of enantiomers using any suitable conventional procedure for resolving enantiomers. Thus, for example, diastereomeric derivatives, e.g. salts, may be produced by reaction of a mixture of enantiomers of formula (IA), e.g. a racemate, and an appropriate chiral compound, e.g. a chiral base. The diastereomers may then be separated by any convenient means, for example by crystallisation, and the desired enantiomer recovered, e.g. by treatment with an acid in the instance where the diastereomer is a salt. In another resolution process a racemate of formula (IA) may be separated using chiral HPLC. Moreover, if desired, a particular enantiomer may be obtained by using an appropriate chiral intermediate in one of the processes described above. Alternatively, a particular enantiomer may be obtained by performing an enantiomer-specific enzymatic biotransformation, e.g. an ester hydrolysis using an esterase, and then purifying only the enantiomerically pure hydrolysed acid from the unreacted ester antipode. Chromatography, recrystallisation and other conventional separation procedures may also be used with intermediates or final products where it is desired to obtain a particular geometric isomer of the invention.

During any of the above synthetic sequences it may be necessary and/or desirable to protect sensitive or reactive groups on any of the molecules concerned. This may be achieved by means of conventional protecting groups, such as those described in Greene's Protective Groups in Organic Synthesis, ed. P.G.M. Wuts, John Wiley & Sons, 5th edition, 2014. The protecting groups may be removed at any convenient subsequent stage utilising methods known from the art.

The compounds in accordance with this invention potently inhibit the ability of IL-17 A to bind to IL-17RA. When tested in the IL-17 FRET assay described below, compounds of the present invention exhibit an IC₅₀ value of 10 µM or less, generally of 5 µM or less, usually of 1 µM or less, typically of 500 nM or less, suitably of 100 nM or less, ideally of 50 nM or less, and preferably of 25 nM or less (the skilled person will appreciate that a *lower* IC₅₀ figure denotes a *more active* compound).

Moreover, certain compounds in accordance with this invention potently inhibit IL-17 induced IL-6 release from human dermal fibroblasts. Indeed, when tested in the HDF cell line assay described below, compounds of the present invention exhibit an IC₅₀ value of 10 µM or less, generally of 5 µM or less, usually of 1 µM or less, typically of 500 nM or less, suitably of 100 nM or less, ideally of 50 nM or less, and preferably of 25 nM or less (as before, the skilled person will appreciate that a *lower* IC₅₀ figure denotes a *more active* compound).

### IL-17 FRET Assay

The purpose of this assay is to test the ability of compounds to disrupt the interaction between IL-17A and soluble IL-17 Receptor A (IL-17RA). The ability of a compound to inhibit IL-17A binding to IL-17RA is measured in this assay.

An IL-17AA-TEV-Human Fc construct was expressed in a CHO SXE cell system and purified by protein A chromatography and size exclusion. The protein was labelled with an amine reactive AlexaFluor 647 dye (Thermo Fisher #A20006), as per manufacturer's instruction.

Soluble IL-17RA (33-317)-HKH-TEV-Fc was expressed in an Expi HEK293 cell system and purified by protein A chromatography and size exclusion. The Fc tag was cleaved by TEV, producing IL-17RA (33-317)-HKH, and the protein was labelled with amine reactive terbium (Thermo Fisher #PV3581).

In assay buffer [Dulbecco's PBS (Sigma #14190-094), 0.05% P20 (Thermo Scientific #28320), 1 mg/mL BSA (Sigma #A2153-500G)] the following solutions were prepared:
For IL-17A assay
- IL-17A-Fc-AF647 at 5 nM
- IL-17RA-HKH-Tb at 5 nM

Compounds were serially diluted in DMSO before receiving an aqueous dilution into a 384 well dilution plate (Greiner #781281), to give a 25% DMSO solution.

IL-17A (10 µL) was added to a black low volume assay plate (Costar #4511) and diluted compound (5 µL) was transferred from the aqueous dilution plate. The cytokine and compound were allowed to incubate for 1 h, then IL-17RA (10 µL) was added. The plates were wrapped in foil and incubated at room temperature for 18-20 h with gentle shaking (<400 rpm) before being read on a Perkin Elmer Envision plate reader (Excitation: 330 nm; Emission 615/645 nm).

The final assay concentrations were IL-17A-AF647 2 nM and IL-17RA-Tb 2 nM, 5% DMSO.

When tested in the IL-17 FRET assay, the compounds of the accompanying Examples were all found to exhibit IC₅₀ values of 10 µM or better.

When tested in the IL-17 FRET assay, compounds of the accompanying Examples exhibit IC₅₀ values generally in the range of about 0.01 nM to about 10 µM, usually in the range of about 0.01 nM to about 5 µM, typically in the range of about 0.01 nM to about 1 µM, suitably in the range of about 0.01 nM to about 500 nM, appositely in the range of about 0.01 nM to about 100 nM, ideally in the range of about 0.01 nM to about 50 nM, and preferably in the range of about 0.01 nM to about 25 nM.

### Inhibition of IL-17A induced IL-6 release from Dermal Fibroblast Cell Line

The purpose of this assay is to test the neutralising ability to IL-17 proteins, in a human primary cell system. Stimulation of normal human dermal fibroblasts (HDF) with IL-17 alone produces only a very weak signal but in combination with certain other cytokines, such as TNFα, a synergistic effect can be seen in the production of inflammatory cytokines, i.e. IL-6.

HDFs were stimulated with IL-17A (50 pM) in combination with TNF-α (25 pM). The resultant IL-6 response was then measured using a homogenous time-resolved FRET kit from Cisbio. The kit utilises two monoclonal antibodies, one labelled with Eu-Cryptate (Donor) and the second with d2 or XL665 (Acceptor). The intensity of the signal is proportional to the concentration of IL-6 present in the sample (Ratio is calculated by 665/620 x 104).

The ability of a compound to inhibit IL-17 induced IL-6 release from human dermal fibroblasts is measured in this assay.

HDF cells (Sigma #106-05n) were cultured in complete media (DMEM + 10% FCS + 2 mM L-glutamine) and maintained in a tissue culture flask using standard techniques. Cells were harvested from the tissue culture flask on the morning of the assay using TrypLE (Invitrogen #12605036). The TrypLE was neutralised using complete medium (45 mL) and the cells were centrifuged at 300 x g for 3 minutes. The cells were re-suspended in complete media (5 mL) counted and adjusted to a concentration of 3.125 × 10⁴ cells/mL before being added to the 384 well assay plate (Corning #3701) at 40 µL per well. The cells were left for a minimum of three hours, at 37°C/5% CO₂, to adhere to the plate.

Compounds were serially diluted in DMSO before receiving an aqueous dilution into a 384 well dilution plate (Greiner #781281), where 5 µL from the titration plate was transferred to 45 µL of complete media and mixed to give a solution containing 10% DMSO.

Mixtures of TNFα and IL-17 cytokine were prepared in complete media to final concentrations of TNFα 25 pM/IL-17A 50 pM, then 30 µL of the solution was added to a 384 well reagent plate (Greiner #781281).

10 µL from the aqueous dilution plate was transferred to the reagent plate containing 30 µL of the diluted cytokines, to give a 2.5% DMSO solution. The compounds were incubated with the cytokine mixtures for one hour at 37°C. After the incubation, 10 µL was transferred to the assay plate, to give a 0.5% DMSO solution, then incubated for 18-20 h at 37°C/5% CO₂.

From the Cisbio IL-6 FRET kit (Cisbio #62IL6PEB) europium cryptate and Alexa 665 were diluted in reconstitution buffer and mixed 1:1, as per kit insert. To a white low volume 384 well plate (Greiner #784075) were added FRET reagents (10 µL), then supernatant (10 µL) was transferred from the assay plate to Greiner reagent plate. The mixture was incubated at room temperature for 3 h with gentle shaking (<400 rpm) before being read on a Synergy Neo 2 plate reader (Excitation: 330 nm; Emission: 615/645 nm).

When tested in the above assay, compounds of the accompanying Examples were found to exhibit IC₅₀ values of 10 µM or better.

When tested in the above assay, compounds of the accompanying Examples exhibit IC₅₀ values generally in the range of about 0.01 nM to about 10 µM, usually in the range of about 0.01 nM to about 5 µM, typically in the range of about 0.01 nM to about 1 µM, suitably in the range of about 0.01 nM to about 500 nM, appositely in the range of about 0.01 nM to about 100 nM, ideally in the range of about 0.01 nM to about 50 nM, and preferably in the range of about 0.01 nM to about 25 nM.

The following Examples illustrate the preparation of compounds according to the invention.

### EXAMPLES

### Abbreviations

| | | | |
|---|---|---|---|
| DCM: | dichloromethane | DMF: | *N,N*-dimethylformamide |
| MeOH: | methanol | THF: | tetrahydrofuran |
| DMSO: | dimethyl sulfoxide | DIPEA: | *N,N*-diisopropylethylamine |
| EtOAc: | ethyl acetate | HOBt: | 1-hydroxybenzotriazole |
| TFA: | trifluoroacetic acid | | |
| EDC.HCl: | *N*-(3-dimethylaminopropyl)-*N*'-ethylcarbodiimide hydrochloride | | |
| SEM-Cl: | 2-(trimethylsilyl)ethoxymethyl chloride | | |
| h: | hour | r.t.: | room temperature |
| M: | mass | RT: | retention time |
| HPLC: | High Performance Liquid Chromatography | | |
| LCMS: | | | Liquid Chromatography Mass Spectrometry |
| ES+: | | | Electrospray Positive Ionisation |

### Analytical Conditions

Compounds were named with the aid of ACD/Name Batch (Network) version 11.01, and/or Accelrys Draw 4.2, and/or Elemental, Dotmatics, and/or Chemaxon.

All reactions involving air- or moisture-sensitive reagents were performed under a nitrogen atmosphere using dried solvents and glassware.

NMR spectra were recorded on a Bruker Avance III HD 500 MHz, 400 MHz, 300 MHz or 250 MHz spectrometer.

### HPLC-MS

**1.** Performed on an Agilent 1200-6120 LC-MS system coupled to Detection (230 to 400 nm and 215 nm) and Mass Spec Detection Agilent 6120 Mass Spectrometer (ES) *m*/*z* 120 to 800.

### Method 1

### Waters X-Bridge C18 (2.1 × 20 mm, 2.5 µm) column

| | |
|---|---|
| Mobile Phase A: | 10 mM ammonium formate in water + 0.1% formic acid |
| Mobile Phase B: | acetonitrile + 5% water + 0.1% formic acid |
| Gradient program: | Flow rate 1 mL/minute |

| Time | A% | B% |
|---|---|---|
| 0.00 | 94.00 | 6.00 |
| 1.50 | 5.00 | 95.00 |
| 2.25 | 5.00 | 95.00 |
| 2.50 | 94.00 | 6.00 |

**2.** Performed on a Shimadzu LCMS-2010EV system coupled to SPD-M20A PDA and PL 2100 detectors.

### Method 2

### Waters Atlantis dC18 (2.1 × 100 mm, 3 µm) column

| | |
|---|---|
| Mobile Phase A: | 0.1% formic acid in water |
| Mobile Phase B: | 0.1% formic acid in acetonitrile |
| Gradient program: | Flow rate 0.6 mL/minute; column temperature 40°C |

| Time | A% | B% |
|---|---|---|
| 0.00 | 95.00 | 5.00 |
| 5.00 | 0.00 | 100.0 |
| 5.40 | 0.00 | 100.0 |
| 5.42 | 95.00 | 5.00 |

### Method 3

### Phenomenex Kinetex Core-Shell C8 (2.1 × 50 mm, 5 µm) column

| | |
|---|---|
| Mobile Phase A: | 0.1% formic acid in water |
| Mobile Phase B: | 0.1% formic acid in acetonitrile |
| Gradient program: | Flow rate 1.2 mL/minute; column temperature 40°C |

| Time | A% | B% |
|---|---|---|
| 0.00 | 95.00 | 5.00 |
| 1.20 | 0.00 | 100.0 |
| 1.30 | 0.00 | 100.0 |
| 1.31 | 95.00 | 5.00 |

**3.** Performed on a Waters ZQ system coupled to Waters 2996 PDA and Waters 2420 detectors.

### Method 4

### Waters Atlantis dC18 (4.6 x 50 mm, 3µm) column

| | |
|---|---|
| Mobile Phase A: | 0.1% formic acid in water |
| Mobile Phase B: | 0.1% formic acid in acetonitrile |
| Gradient program: | Flow rate 0.8 mL/minute; column temperature 40°C |

| Time | A% | B% |
|---|---|---|
| 0.00 | 30.00 | 70.00 |
| 3.00 | 90.00 | 10.0 |
| 6.00 | 90.00 | 10.0 |

### Method 5

### Waters Atlantis dC18 (4.6 x 50 mm, 3 µm) column

| | |
|---|---|
| Mobile Phase A: | 0.1% formic acid in water |
| Mobile Phase B: | 0.1% formic acid in acetonitrile |
| Gradient program: | Flow rate 0.6 mL/minute; column temperature 40°C |

| Time | A% | B% |
|---|---|---|
| 0.00 | 50.00 | 50.00 |
| 3.00 | 95.00 | 5.00 |
| 6.00 | 95.00 | 5.00 |

**4.** Performed on a Shimadzu LCMS-2010EV system coupled to SPD-M20A PDA and Softa Model 400 ELS detectors

### Method 6

### Waters X-Bridge C18 (2.1 × 30 mm, 2.5 µm) column

| | |
|---|---|
| Mobile Phase A: | 5 mM ammonium formate in water + 0.1% ammonia solution |
| Mobile Phase B: | acetonitrile + 5% 5 mM ammonium formate in water + 0.1% ammonia solution |
| Gradient program: | Flow rate 1 mL/minute |

| Time | A% | B% |
|---|---|---|
| 0.00 | 95.00 | 5.00 |
| 4.00 | 5.00 | 95.00 |
| 5.00 | 5.00 | 95.00 |
| 5.10 | 95.00 | 5.00 |
| 6.50 | 95.00 | 5.00 |

### INTERMEDIATE 1

### Methyl 2-cyclooctylidene-2-formamidoacetate

A solution of potassium *tert*-butoxide in THF (1M, 48 mL, 48 mmol) was added dropwise to a solution of methyl isocyanoacetate (4.0 mL, 41.8 mmol) in anhydrous THF (40 mL) at -65°C under nitrogen. After 5 minutes, a solution of cyclooctanone (5 g, 39.62 mmol) in anhydrous THF (20 mL) was added slowly at -70°C. The reaction mixture was stirred at -70°C for 30 minutes, then allowed to warm to r.t. After 60 h, the reaction mixture was quenched with water (100 mL) and stirred at r.t. for 1 h. The residue was extracted with ethyl acetate (3 x 100 mL). The combined organic extracts were washed with brine (50 mL) and dried over magnesium sulfate, then filtered and concentrated *in vacuo.* The residue was purified by flash column chromatography, using a gradient of ethyl acetate in heptane (0-90%), to afford the *title compound* (5.37 g, 58%) as a viscous orange oil, which solidified upon standing. δ_{H} (500 MHz, DMSO-d₆) 9.31 (s, 1H), 8.01 (d, *J* 1.5 Hz, 1H), 3.60 (s, 3H), 2.52-2.47 (m, 2H), 2.31-2.23 (m, 2H), 1.74-1.60 (m, 4H), 1.50-1.31 (m, 6H). HPLC-MS (method 1): MNa+ *m*/*z* 248, RT 1.63 minutes.

### INTERMEDIATE 2

### Methyl 2-cyclooctyl-2-formamidoacetate

Magnesium turnings (3.15 g, 130 mmol) were added to a stirred solution of *Intermediate 1* (3.04 g, 13.00 mmol) in anhydrous MeOH (65 mL) at 0°C. The suspension was stirred at 0°C for 1 h, then allowed to warm to r.t. After 16 h, an additional portion of magnesium turnings (1.00 g, 41.1 mmol) was added, and the suspension was stirred at r.t. for 3.5 h. The mixture was concentrated *in vacuo.* The residue was partitioned between EtOAc (100 mL) and water (200 mL), then cooled to 0°C. Concentrated HCl was cautiously added to aid dissolution of the solids (pH 4). The organic layer was removed, then the aqueous suspension was treated with further concentrated HCl (pH 1) and the material was extracted with EtOAc (2 x 100 mL). The combined organic extracts were washed with brine (50 mL) and dried over magnesium sulfate, then filtered and concentrated *in vacuo.* The residue was purified by flash column chromatography, using a gradient of EtOAc in heptane (0-80%), to afford the *title compound* (1.53 g, 52%) as a viscous orange oil. δ_{H} (500 MHz, DMSO-d₆) 8.46 (d, *J* 8.5 Hz, 1H), 8.06 (s, 1H), 4.29 (dd, J 8.6, 6.1 Hz, 1H), 3.64 (s, 3H), 2.04-1.93 (m, 1H), 1.73-1.19 (m, 14H). HPLC-MS (method 2): MH+ *m*/*z* 228, RT 3.94 minutes.

### INTERMEDIATE 3

### Methyl 2-amino-2-cyclooctylacetate hydrochloride

Acetyl chloride (1.9 mL, 26.7 mmol) was added at 0°C to a stirred solution of *Intermediate 2* (1.69 g, 6.77 mmol) in MeOH (70 mL) under nitrogen. The mixture was heated at 50°C for 2 h, then cooled to r.t. and concentrated *in vacuo.* The resulting crude material was triturated with diethyl ether (40 mL) and the solids were collected by filtration, washing with diethyl ether (2 x 20 mL). The solids were dried *in vacuo* at 50°C for 6 h to afford the *title compound* (1.43 g, 81%) as a tan powder. δ_{H} (500 MHz, DMSO-d₆) 8.61 (br s, 3H), 3.86 (d, J 4.4 Hz, 1H), 3.73 (s, 3H), 2.19-2.09 (m, 1H), 1.68-1.37 (m, 13H), 1.32-1.20 (m, 1H). HPLC-MS (method 3): MH+ *m*/*z* 200, RT 0.75 and 0.86 minutes.

### INTERMEDIATE 4

### Methyl 2-cyclooctyl-2-[(3-methylisoxazole-4-carbonyl)amino]acetate

To a solution of 3-methylisoxazole-4-carboxylic acid (12.9 g, 66.1 mmol) in dry DMF (100 mL) at 0°C were added DIPEA (54.9 g, 424.6 mmol), EDC.HCl (19.5 g, 101.9 mmol) and HOBt (13.8 g, 101.9 mmol). The reaction mixture was stirred for 15 minutes at 0°C, then *Intermediate 3* (20.0 g, 84.9 mmol) was added. The reaction mixture was stirred at r.t. for 48 h, then poured into ice-cold water (500 mL) and extracted with EtOAc (2 x 400 mL). The organic layer was separated, washed with ice-cold water (2 x 100 mL) and 1N HCl (2 × 50 mL), then dried over anhydrous Na₂SO₄, filtered and evaporated *in vacuo.* The residue was purified by flash column chromatography, using a gradient of EtOAc in hexanes (0-15%), to afford the *title compound* (7.9 g, 41.3%) as a pale yellow viscous oil. LC-MS (method 4): MH+ *m*/*z* 309, RT 5.5 minutes.

### INTERMEDIATE 5

### Lithium 2-cyclooctyl-2-[(3-methylisoxazole-4-carbonyl)amino]acetate

To a solution of *Intermediate 4* (11.01 g, 35.7 mmol) in THF (90 mL) were added water (30 mL) and lithium hydroxide monohydrate (2.25 g, 53.6 mmol). The reaction mixture was stirred at r.t. for 16 h, then concentrated *in vacuo.* To the residue was added diethyl ether (50 mL). The mixture was stirred for 10 minutes, then filtered. The resultant solid was washed with diethyl ether (50 mL) and pentane (50 mL), then dried under vacuum, to afford the *title compound* (9.51 g, 91%) as an off-white solid. δ_{H} (400 MHz, DMSO-d₆) 9.69 (s, 1H), 8.21 (s, 1H), 4.11 (dd, J 8.0, 4.0 Hz, 1H), 2.35 (s, 3H), 2.05 (br s, 1H), 1.65-1.35 (m, 14H). HPLC-MS (method 5): MH+ *m*/*z* 295, RT 5.4 minutes.

### INTERMEDIATE 6

### N-(4-Bromo-2-nitrophenyl)oxetan-3-amine

To a stirred solution of 4-bromo-1-fluoro-2-nitrobenzene (2.00 g, 9.09 mmol) in THF (50 mL) were added oxetan-3-amine (0.80 g, 10.9 mmol) and potassium carbonate (1.26 g, 9.09 mmol) at 0°C. The reaction mixture was heated in a sealed tube at 60°C for 6 h, then diluted with EtOAc (500 mL) and washed with water (2 x 200 mL). The organic layer was separated and dried over sodium sulfate, then filtered and concentrated *in vacuo.* The residue was purified by recrystallisation from 5% EtOAc in hexanes (100 mL) to afford the *title compound* (2.20 g, 86%) as a yellow solid. δ_{H} (400 MHz, DMSO-d₆) 8.26 (d, *J* 3.9 Hz, 1H), 8.19 (d, *J* 2.0 Hz, 1H), 7.65 (dd, *J* 9.1, 2.2 Hz, 1H), 6.69 (d, *J* 9.3 Hz, 1H), 4.92-4.86 (m, 2H), 4.84-4.77 (m, 1H), 4.59-4.54 (m, 2H). HPLC-MS (method 6): no ionisation, RT 1.97 minutes.

### INTERMEDIATE 7

### 4-Bromo-N¹-(oxetan-3-yl)benzene-1,2-diamine

To a solution of *Intermediate* 6 (2.20 g, 8.06 mmol) in MeOH (100 mL) were added zinc (2.64 g, 40.3 mmol) and ammonium formate (2.54 g, 40.3 mmol) at 0°C. The reaction mixture was allowed to warm to r.t. with stirring. After 1 h, the reaction mixture was filtered through a pad of Celite^{®}, washing with MeOH (3 x 50 mL), and the filtrate was concentrated *in vacuo.* The residue was dissolved in DCM (500 mL), cooled to 0°C over 15 minutes, then decanted and washed with water (2 x 200 mL). The organic layer was separated and dried over sodium sulfate, then filtered and concentrated *in vacuo.* The residue was purified by recrystallisation from 5% EtOAc in hexanes (100 mL) to afford the *title compound* (1.60 g, 81%) as a brown solid. δ_{H} (400 MHz, DMSO-d₆) 6.69 (s, 1H), 6.54 (d, *J* 8.3 Hz, 1H), 6.02 (d, *J* 8.3 Hz, 1H), 5.27 (d, *J* 5.4 Hz, 1H), 4.92 (br s, 2H), 4.89-4.82 (m, 2H), 4.50-4.38 (m, 3H). HPLC-MS (method 6): MH+ *m*/*z* 243, RT 1.74 minutes.

### INTERMEDIATE 8

### 6-Bromo-3-(oxetan-3-yl)-1H-benzimidazol-2-one

To a solution of *Intermediate 7* (1.60 g, 6.58 mmol) in THF (50 mL) was added 1,1'-carbonyldiimidazole (1.60 g, 9.87 mmol) at 0°C. The reaction mixture was stirred at r.t. for 6 h, then quenched with water (100 mL) and extracted with a 10% solution of MeOH in DCM (2 x 200 mL). The organic layer was separated and washed with brine (100 mL), then dried over sodium sulfate, filtered and concentrated *in vacuo.* The residue was purified by recrystallisation from 20% EtOAc in hexanes (50 mL) to afford the *title compound* (1.40 g, 77%) as a light pink solid. δ_{H} (400 MHz, DMSO-d₆) 11.14 (br s, 1H), 7.38 (d, *J* 8.3 Hz, 1H), 7.24 (dd, *J* 8.6, 1.7 Hz, 1H), 7.16 (d, *J* 2.0 Hz, 1H), 5.49-5.41 (m, 1H), 5.04-5.00 (m, 2H), 4.97-4.90 (m, 2H). HPLC-MS (method 6): M⁺ *m*/*z* 269, RT 1.66 minutes.

### INTERMEDIATE 9

### 5-Bromo-1-(oxetan-3-yl)-3-[2-(trimethylsilyl)ethoxymethyl]benzimidazol-2-one

To a stirred solution of *Intermediate 8* (1.40 g, 5.20 mmol) in DMF (30 mL) was added sodium hydride (0.19 g, 7.80 mmol) at 0°C. The reaction mixture was stirred at 0°C for 30 minutes. SEM-C1 (1.74 g, 10.4 mmol) was added at 0°C, and the reaction mixture was allowed to warm to r.t. After 3 h, the reaction mixture was quenched with water (50 mL) and extracted with EtOAc (2 x 200 mL). The organic layer was separated, washed with water (200 mL) and brine (100 mL), then dried over sodium sulfate, filtered and concentrated *in vacuo.* The residue was purified by flash column chromatography, using a gradient of EtOAc in hexanes (20-30%), to afford the *title compound* (1.40 g, 66%) as a light pink solid. δ_{H} (400 MHz, DMSO-d₆) 7.51 (s, 1H), 7.45 (d, *J* 8.8 Hz, 1H), 7.34 (dd, *J* 8.3, 1.0 Hz, 1H), 5.51 (t, *J* 6.9 Hz, 1H), 5.26 (s, 2H), 5.06-5.01 (m, 2H), 4.98-4.92 (m, 2H), 3.55 (t, *J* 8.1 Hz, 2H), 0.89-0.83 (m, 2H), -0.07 (s, 9H). HPLC-MS (method 6): MH+ *m*/*z* 401, RT 2.20 minutes.

### INTERMEDIATE 10

### 5-Amino-1-(oxetan-3-yl)-3-[2-(trimethylsilyl)ethoxymethyl]benzimidazol-2-one

To a solution of *Intermediate 9* (0.40 g, 1.00 mmol) in DMSO (20 mL) were added sodium azide (0.13 g, 2.00 mmol) and potassium carbonate (0.42 g, 3.00 mmol) at r.t. The reaction mixture was purged with argon for 30 minutes. Copper(I) iodide (0.02 g, 0.10 mmol) and L-proline (0.12 g, 1.00 mmol) were added, and the reaction mixture was again purged with nitrogen for 10 minutes. The reaction mixture was heated at 100°C for 16 h, then diluted with EtOAc (400 mL) and washed with water (2 x 100 mL) and brine (100 mL). The organic layer was separated and dried over sodium sulfate, then filtered and concentrated *in vacuo.* The residue was purified by flash column chromatography, using a gradient of EtOAc in hexanes (50-60%), to afford the *title compound* (0.25 g, 57%) as a light yellow solid. δ_{H} (400 MHz, DMSO-d₆) 7.17 (d, *J* 8.3 Hz, 1H), 6.51 (d, *J* 2.0 Hz, 1H), 6.39 (dd, *J* 8.3, 2.0 Hz, 1H), 5.47-5.38 (m, 1H), 5.12 (s, 2H), 5.04-5.00 (m, 2H), 4.96-4.90 (m, 4H), 3.57-3.48 (m, 2H), 0.88-0.79 (m, 2H), -0.05 (s, 9H). HPLC-MS (method 6): MH+ *m*/*z* 336, RT 1.82 minutes.

### INTERMEDIATE 11

### N-[1-Cyclooctyl-2-({1-(oxetan-3-yl)-2-oxo-3-[2-(trimethylsilyl)ethoxymethyl]-benzimidazol-5-yl}amino)-2-oxoethyl]-3-methylisoxazole-4-carboxamide

To a solution of *Intermediate 10* (0.25 g, 0.75 mmol) and *Intermediate 5* (0.22 g, 0.75 mmol) in DCM (20 mL) was added propylphosphonic anhydride (50% solution in EtOAc, 1.42 g, 4.47 mmol) at 0°C. The reaction mixture was stirred at r.t. for 12 h, then diluted with DCM (200 mL) and washed with water (50 mL). The organic layer was separated and dried over sodium sulfate, then filtered and concentrated *in vacuo.* The residue was purified by flash column chromatography, using a gradient of EtOAc in hexanes (50-60%), to afford the *title compound* (0.25 g, 49%) as an off-white solid. δ_{H} (400 MHz, DMSO-d₆) 10.28 (s, 1H), 9.44 (s, 1H), 8.47 (d, *J* 8.8 Hz, 1H), 7.77 (s, 1H), 7.45-7.42 (m, 1H), 7.37-7.31 (m, 1H), 5.53-5.46 (m, 1H), 5.20 (s, 2H), 5.07-5.01 (m, 2H), 4.98-4.91 (m, 2H), 4.51-4.46 (m, 1H), 3.53 (t, *J* 8.1 Hz, 2H), 2.37 (s, 3H), 2.09-2.07 (m, 1H), 1.73-1.37 (m, 14H), 0.84 (t, *J* 7.8 Hz, 2H), -0.08 (s, 9H). HPLC-MS (method 6): MH+ *m*/*z* 612, RT 2.29 minutes.

### INTERMEDIATE 12

### N-(4-Bromo-2-nitrophenyl)tetrahydropyran-4-amine

To a stirred solution of 4-bromo-1-fluoro-2-nitrobenzene (2.00 g, 9.09 mmol) in THF (50 mL) were added tetrahydropyran-4-amine (1.10 g, 10.9 mmol) and potassium carbonate (1.26 g, 9.09 mmol) at 0°C. The reaction mixture was heated in a sealed tube at 60°C for 6 h, then diluted with EtOAc (500 mL) and washed with water (2 x 200 mL). The organic layer was separated and dried over sodium sulfate, then filtered and concentrated *in vacuo.* The residue was purified by flash column chromatography, using a gradient of EtOAc in hexanes (0-15%), to afford the *title compound* (2.70 g, 96%) as an off-white solid. δ_{H} (400 MHz, DMSO-d₆) 8.18 (d, *J* 2.5 Hz, 1H), 7.92 (d, *J* 7.4 Hz, 1H), 7.65 (dd, *J* 9.4, 2.5 Hz, 1H), 7.20 (d, *J* 9.4 Hz, 1H), 3.94-3.82 (m, 3H), 3.50-3.44 (m, 2H), 1.93 (d, *J* 12.8 Hz, 2H), 1.64-1.50 (m, 2H). HPLC-MS (method 6): no ionisation, RT 1.89 minutes.

### INTERMEDIATE 13

### 4-Bromo-N¹-(tetrahydropyran-4-yl)benzene-1,2-diamine

To a solution of *Intermediate 12* (2.00 g, 6.64 mmol) in MeOH (35 mL) were added ammonium formate (2.09 g, 33.2 mmol) and zinc (2.17 g, 33.2 mmol) at 0°C. The reaction mixture was allowed to warm to r.t. with stirring. After 1 h, the reaction mixture was filtered through a pad of Celite^{®}, washing with MeOH (3 x 50 mL), and the filtrate was concentrated *in vacuo.* The residue was diluted with water (100 mL) and extracted with EtOAc (3 x 80 mL). The organic layer was separated and dried over sodium sulfate, the filtered and concentrated *in vacuo,* to afford the *title compound* (1.71 g, 95%) as a pale brown solid, which was utilised without further purification. δ_{H} (400 MHz, DMSO-d₆) 6.67 (d, *J* 2.5 Hz, 1H), 6.56 (dd, *J* 8.3, 2.5 Hz, 1H), 6.41 (d, *J* 8.3 Hz, 1H), 4.85 (s, 2H), 4.33 (d, *J* 7.8 Hz, 1H), 3.90-3.83 (m, 2H), 3.45-3.36 (m, 3H), 1.88 (d, *J* 12.7 Hz, 2H), 1.44-1.33 (m, 2H). HPLC-MS (method 6): MH+ *m*/*z* 273, RT 1.83 minutes.

### INTERMEDIATE 14

### 6-Bromo-3-(tetrahydrot)yran-4-yl)-1H-bezimidazol-2-one

To a solution of *Intermediate 13* (1.70 g, 6.27 mmol) in THF (50 mL) was added 1,1'-carbonyldiimidazole (1.53 g, 9.40 mmol) at 0°C. The reaction mixture was stirred at r.t. for 6 h, then quenched with water (100 mL) and extracted with EtOAc (3 x 100 mL). The organic layer was separated and washed with brine (100 mL), then dried over sodium sulfate, filtered and concentrated *in vacuo.* The residue was purified by flash column chromatography, using a gradient of EtOAc in hexanes (80-90%), to afford the *title compound* (1.71 g, 92%) as an off-white solid. δ_{H} (400 MHz, DMSO-d₆) 11.04 (s, 1H), 7.26-7.22 (m, 1H), 7.17-7.13 (m, 1H), 7.11 (d, *J* 2.0 Hz, 1H), 4.43-4.34 (m, 1H), 3.97 (dd, *J* 11.5, 4.2 Hz, 2H), 3.45 (t, *J* 11.5 Hz, 2H), 2.40-2.29 (m, 2H), 1.68-1.58 (m, 2H). HPLC-MS (method 6): MH+ *m*/*z* 299, RT 1.61 minutes.

### INTERMEDIATE 15

### 5-Bromo-1-(tetrahydropyran-4-yl)-3-[2-(trimethylsilyl)ethoxymethyl]benzimidazol-2-one

To a stirred solution of *Intermediate 14* (1.70 g, 5.72 mmol) in DMF (20 mL) was added sodium hydride (0.34 g, 8.58 mmol) at 0°C. The reaction mixture was stirred at 0°C for 30 minutes. SEM-C1 (1.33 mL, 11.4 mmol) was added at 0°C, and the reaction mixture was allowed to warm to r.t. After 2 h, the reaction mixture was quenched with ice water (150 mL) and filtered, then washed with water (50 mL) and dried *in vacuo,* to afford the *title compound* (2.01 g crude) as an off-white solid, which was utilised without further purification. δ_{H} (400 MHz, DMSO-d₆) 7.45 (d, *J* 1.5 Hz, 1H), 7.35-7.31 (m, 1H), 7.28-7.22 (m, 1H), 5.25 (s, 2H), 4.48-4.39 (m, 1H), 3.98 (dd, *J* 11.5, 4.2 Hz, 2H), 3.56-3.42 (m, 4H), 2.37-2.33 (m, 2H), 1.67-1.60 (m, 2H), 0.83 (t, *J* 8.1 Hz, 2H), -0.09 (s, 9H). HPLC-MS (method 6): no ionisation, RT 2.25 minutes.

### INTERMEDIATE 16

### 5-Amino-1-(tetrahydropyran-4-yl)-3-[2-(trimethylsilyl)ethoxymethyl]benzimidazol-2-one

To a solution of *Intermediate 15* (0.50 g, 1.17 mmol) in DMSO (10 mL) were added sodium azide (0.23 g, 3.51 mmol) and potassium carbonate (0.65 g, 4.68 mmol) at r.t. The reaction mixture was purged with argon for 30 minutes. Copper(I) iodide (0.22 g, 1.17 mmol) and L-proline (0.27 g, 2.34 mmol) were added. The reaction mixture was heated at 120°C for 24 h, then diluted with water (80 mL) and extracted with EtOAc (3 x 50 mL). The organic layer was separated and washed with brine (50 mL), then dried over sodium sulfate, filtered and concentrated *in vacuo.* The residue was purified by flash column chromatography, using a gradient of EtOAc in hexanes (35-45%), to afford the *title compound* (203 mg, 48%) as a pale brown semi-solid. δ_{H} (400 MHz, DMSO-d₆) 6.97 (d, *J* 8.3 Hz, 1H), 6.46 (d, *J* 1.5 Hz, 1H), 6.32 (dd, *J* 8.3, 2.0 Hz, 1H), 5.11 (s, 2H), 4.84 (br s, 2H), 4.30-4.36 (m, 1H), 3.93-4.00 (m, 2H), 3.40-3.54 (m, 4H), 2.27-2.37 (m, 2H), 1.57-1.61 (m, 2H), 0.82-0.88 (m, 2H), -0.02 (s, 9H). HPLC-MS (method 6): MH+ *m*/*z* 364, RT 1.92 minutes.

### INTERMEDIATE 17

### N-[1-Cyclooctyl-2-oxo-2-({2-oxo-1-(tetrahydrotpyran-4-yl)-3-[2-(trimethylsilyl)ethoxymethyl]benzimidazol-5-yl}amino)ethyl]-3-methylisoxazole-4-carboxamide

To a solution of *Intermediate 16* (0.20 g, 0.55 mmol) and *Intermediate 5* (0.16 g, 0.55 mmol) in DCM (20 mL) was added propylphosphonic anhydride (50% solution in EtOAc, 1.05 g, 3.30 mmol) at 0°C. The reaction mixture was stirred at r.t. for 12 h, then diluted with DCM (100 mL) and washed with water (50 mL). The organic layer was separated and dried over sodium sulfate, then filtered and concentrated *in vacuo.* The residue was purified by flash column chromatography, using a gradient of EtOAc in hexanes (50-60%), to afford the *title compound* (0.23 g, 65%) as an off-white solid. δ_{H} (400 MHz, DMSO-d₆) 10.20 (s, 1H), 9.44 (s, 1H), 8.44 (d, *J* 8.8 Hz, 1H), 7.68 (s, 1H), 7.28 (s, 2H), 5.19 (s, 2H), 4.49-4.41 (m, 2H), 4.04-3.93 (m, 3H), 3.54-3.43 (m, 4H), 2.37 (s, 3H), 2.09-2.06 (m, 2H), 1.73-1.36 (m, 16H), 0.87-0.79 (m, 2H), -0.10 (s, 9H). HPLC-MS (method 6): M⁺ *m*/*z* 639, RT 2.26 minutes.

### EXAMPLE 1

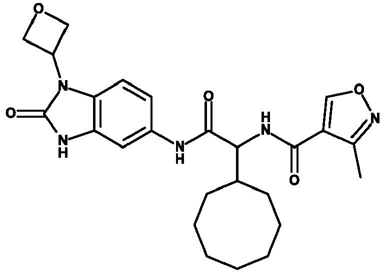

### N-(1-Cyclooctyl-2-{[1-(oxetan-3-yl)-2-oxo-3H-benzimidazol-5-yl]amino}-2-oxoethyl)-3-methylisoxazole-4-carboxamide

To a solution of *Intermediate 11* (0.16 g, 0.26 mmol) in DCM (20 mL) was added TFA (1.00 mL, 13.1 mmol) at 0°C. The reaction mixture was stirred at r.t. for 30 minutes, then concentrated *in vacuo.* The residue was diluted with acetonitrile (2 mL) and methanolic ammonia (3 mL) at 0°C and stirred at r.t. for 10 minutes, then concentrated *in vacuo.* The residue was purified by flash column chromatography, using a gradient of MeOH in DCM (3-4%), to afford the *title compound* (35 mg, 27%) as a white solid. δ_{H} (400 MHz, DMSO-d₆) 10.92 (s, 1H), 10.18 (s, 1H), 9.43 (s, 1H), 8.47 (d, *J* 8.8 Hz, 1H), 7.59 (s, 1H), 7.36 (d, *J* 8.8 Hz, 1H), 7.19 (d, *J* 8.3 Hz, 1H), 5.43 (t, J 7.1 Hz, 1H), 5.04 (t, *J* 6.4 Hz, 2H), 4.96-4.91 (m, 2H), 4.46 (t, *J* 8.8 Hz, 1H), 2.37 (s, 3H), 2.09-2.07 (m, 1H), 1.65-1.36 (m, 14H). HPLC-MS (method 6): MH+ *m*/*z* 482, RT 2.52 minutes.

### EXAMPLE 2

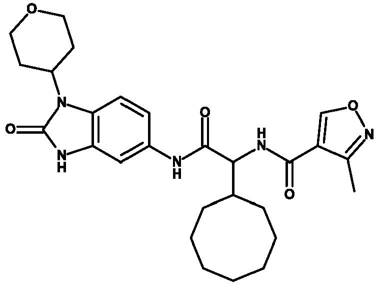

### N-(1-Cyclooctyl-2-oxo-2-{[2-oxo-1-(tetrahydropyran-4-yl)-3H-benzimidazol-5-yl]-amino}ethyl)-3-methylisoxazole-4-carboxamide

To a solution of *Intermediate 17* (0.20 g, 0.31 mmol) in DCM (5 mL) was added TFA (0.96 mL, 12.5 mmol) at 0°C. The reaction mixture was stirred at r.t. for 3 h, then concentrated *in vacuo.* The residue was diluted with acetonitrile (1 mL) and methanolic ammonia (2 mL) at 0°C and stirred at r.t. for 1 h, then concentrated *in vacuo.* The residue was purified by flash column chromatography, using a gradient of MeOH in DCM (3-4%), to afford the *title compound* (9 mg, 57%) as a white solid. δ_{H} (400 MHz, DMSO-d₆) 10.81 (s, 1H), 10.13 (s, 1H), 9.43 (s, 1H), 8.47 (d, *J* 8.8 Hz, 1H), 7.52 (s, 1H), 7.20-7.16 (m, 1H), 7.14-7.10 (m, 1H), 4.45 (t, *J* 8.8 Hz, 1H), 4.40-4.30 (m, 1H), 3.98 (dd, *J* 11.0, 3.7 Hz, 2H), 3.45 (t, *J* 11.5 Hz, 2H), 2.37 (s, 3H), 2.35-2.31 (m, 2H), 2.13-2.07 (m, 1H), 1.72-1.35 (m, 16H). HPLC-MS (method 6): MH+ *m*/*z* 510, RT 2.60 minutes.

## Claims

1. A compound of formula (IA) or an *N*-oxide thereof, or a pharmaceutically acceptable salt thereof: 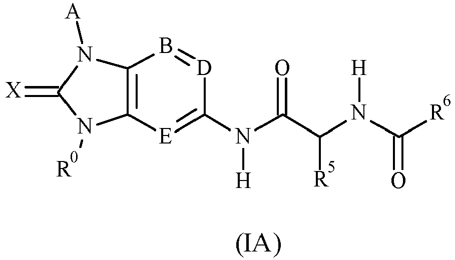 wherein
A represents oxetanyl or tetrahydropyranyl, either of which groups may be optionally substituted by one, two or three substituents independently selected from C₁₋₆ alkyl, oxo and imino;
B represents C-R²;
D represents C-R³;
E represents C-R⁴;
X represents O or S;
R⁰ represents hydrogen or Cₗ-₆ alkyl;
R² represents hydrogen or halogen;
R³ represents hydrogen or halogen;
R⁴ represents hydrogen;
R⁵ represents C₃₋₉ cycloalkyl, which group may be optionally substituted by one, two or three substituents independently selected from halogen, cyano, C₁₋₆ alkyl, trifluoromethyl, phenyl, hydroxy, C₁₋₆ alkoxy and aminocarbonyl; and
R⁶ represents heteroaryl, which group may be optionally substituted by one, two or three substituents independently selected from C₁₋₆ alkyl.

2. A compound as claimed in claim 1 represented by formula (IIA), or a pharmaceutically acceptable salt thereof: 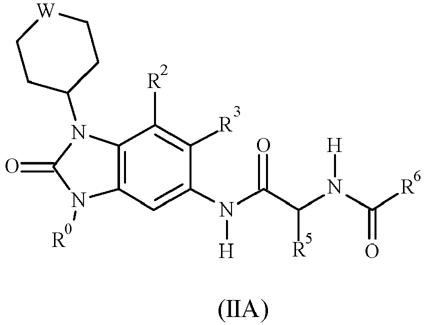 wherein
W represents O; and
R⁰, R², R³, R⁵ and R⁶ are as defined in claim 1.

3. A compound as claimed in claim 1 represented by formula (IIB), or a pharmaceutically acceptable salt thereof: 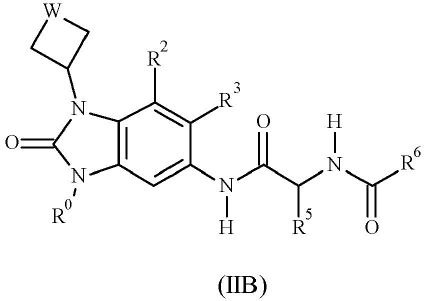 wherein
W represents O; and
R⁰, R², R³, R⁵ and R⁶ are as defined in claim 1.

4. A compound as claimed in claim 1 selected from the following:
*N-*(1-cyclooctyl-2-{[1-(oxetan-3-yl)-2-oxo-3*H*-benzimidazol-5-yl]amino}-2-oxoethyl)-3-methylisoxazole-4-carboxamide; and
*N*-(1-cyclooctyl-2-oxo-2-{[2-oxo-1-(tetrahydropyran-4-yl)-3*H*-benzimidazol-5-yl]-amino}ethyl)-3-methylisoxazole-4-carboxamide.

5. A compound of formula (IA) as defined in claim 1 or an *N*-oxide thereof, or a pharmaceutically acceptable salt thereof, for use in therapy.

6. A compound of formula (IA) as defined in claim 1 or an *N*-oxide thereof, or a pharmaceutically acceptable salt thereof, for use in the treatment and/or prevention of an inflammatory or autoimmune disorder.

7. A pharmaceutical composition comprising a compound of formula (IA) as defined in claim 1 or an *N*-oxide thereof, or a pharmaceutically acceptable salt thereof, in association with a pharmaceutically acceptable carrier.

8. A pharmaceutical composition as claimed in claim 7 further comprising an additional pharmaceutically active ingredient.

## Patentansprüche

1. Verbindung der Formel (IA) oder ein *N*-Oxid davon oder ein pharmazeutisch unbedenkliches Salz davon: 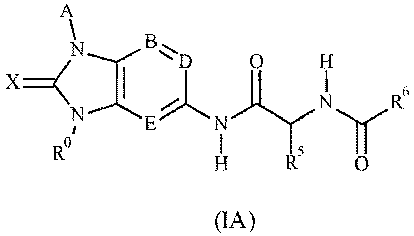 wobei
A für Oxetanyl oder Tetrahydropyranyl steht, wobei jede dieser Gruppen gegebenenfalls durch einen, zwei oder drei Substituenten, die unabhängig aus C₁₋₆-Alkyl, Oxo und Imino ausgewählt sind, substituiert sein kann;
B für C-R² steht;
D für C-R³ steht;
E für C-R⁴ steht;
X für O oder S steht;
R° für Wasserstoff oder C₁₋₆-Alkyl steht;
R² für Wasserstoff oder Halogen steht;
R³ für Wasserstoff oder Halogen steht;
R⁴ für Wasserstoff steht;
R⁵ für C₃₋₉-Cycloalkyl steht, wobei diese Gruppe gegebenenfalls durch einen, zwei oder drei Substituenten, die unabhängig aus Halogen, Cyano, C₁₋₆-Alkyl, Trifluormethyl, Phenyl, Hydroxy, C₁₋₆-Alkoxy und Aminocarbonyl ausgewählt sind, substituiert sein kann; und
R⁶ für Heteroaryl steht, wobei diese Gruppe gegebenenfalls durch einen, zwei oder drei Substituenten, die unabhängig aus C₁₋₆-Alkyl ausgewählt sind, substituiert sein kann.

2. Verbindung nach Anspruch 1, die durch Formel (IIA) wiedergegeben wird, oder ein pharmazeutisch unbedenkliches Salz davon: 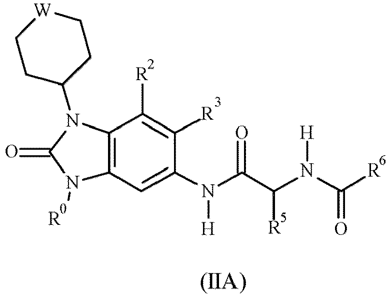 wobei
W für O steht und
R⁰, R², R³, R⁵ und R⁶ wie in Anspruch 1 definiert sind.

3. Verbindung nach Anspruch 1, die durch Formel (IIB) wiedergegeben wird, oder ein pharmazeutisch unbedenkliches Salz davon: 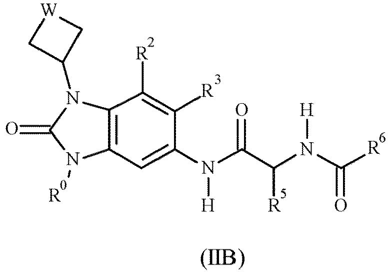 wobei
W für O steht und
R⁰, R², R³, R⁵ und R⁶ wie in Anspruch 1 definiert sind.

4. Verbindung nach Anspruch 1, ausgewählt aus den Folgenden:
*N*-(1-Cyclooctyl-2-{[1-(oxetan-3-yl)-2-oxo-3*H-*benzimidazolyl-5-yl]amino}-2-oxoethyl)-3-methylisoxazol-4-carboxamid und
*N*-(1-Cyclooctyl-2-oxo-2-{[2-oxo-1-(tetrahydropyran-4-yl)-3*H*-benzimidazolyl-5-yl]amino}ethyl)-3-methylisoxazol-4-carboxamid.

5. Verbindung der Formel (IA) gemäß Anspruch 1 oder ein N-Oxid davon oder ein pharmazeutisch unbedenkliches Salz davon zur Verwendung bei der Therapie.

6. Verbindung der Formel (IA) gemäß Anspruch 1 oder ein N-Oxid davon oder ein pharmazeutisch unbedenkliches Salz davon zur Verwendung bei der Behandlung und/oder Prävention einer entzündlichen Störung oder Autoimmunstörung.

7. Pharmazeutische Zusammensetzung, umfassend eine Verbindung der Formel (IA) gemäß Anspruch 1 oder ein N-Oxid davon oder ein pharmazeutisch unbedenkliches Salz davon in Assoziation mit einem pharmazeutisch unbedenklichen Träger.

8. Pharmazeutische Zusammensetzung nach Anspruch 7, die ferner einen zusätzlichen pharmazeutischen Wirkstoff umfasst.

## Revendications

1. Composé de formule (IA) ou *N*-oxyde correspondant ou sel pharmaceutiquement acceptable correspondant : 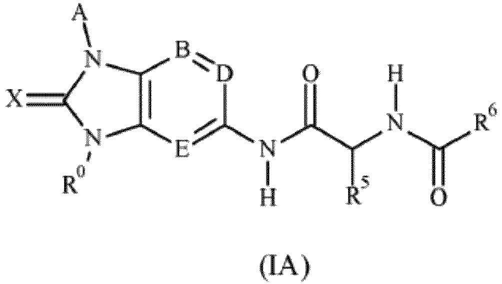
A représentant oxétanyle ou tétrahydropyrannyle, l'un ou l'autre desquels groupes pouvant éventuellement être substitué par un, deux ou trois substituants indépendamment choisis parmi alkyle en C₁₋₆, oxo et imino ;
B représentant C-R² ;
D représentant C-R³ ;
E représentant C-R⁴ ;
X représentant O ou S ;
R° représentant hydrogène ou alkyle en C₁₋₆ ;
R² représentant hydrogène ou halogène ;
R³ représentant hydrogène ou halogène ;
R⁴ représentant hydrogène ;
R⁵ représentant cycloalkyle en C₃₋₉, lequel groupe pouvant éventuellement être substitué par un, deux ou trois substituants indépendamment choisis parmi halogène, cyano, alkyle en C₁₋₆, trifluorométhyle, phényle, hydroxy, alcoxy en C₁₋₆ et aminocarbonyle ; et
R⁶ représentant hétéroaryle, lequel groupe pouvant éventuellement être substitué par un, deux ou trois substituants indépendamment choisis parmi alkyle en C₁₋₆.

2. Composé selon la revendication 1 représenté par la formule (IIA), ou sel pharmaceutiquement acceptable correspondant : 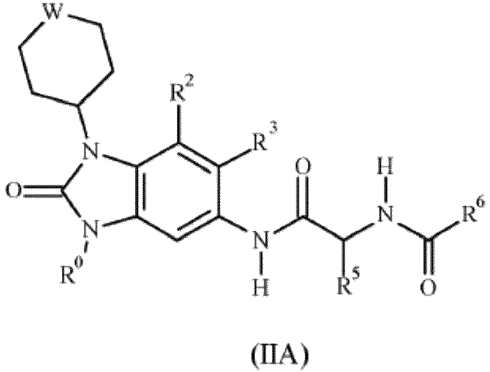
W représentant O ; et
R⁰, R², R³, R⁴, R⁵ et R⁶ étant tels que définis dans la revendication 1.

3. Composé selon la revendication 1 représenté par la formule (IIB), ou sel pharmaceutiquement acceptable correspondant : 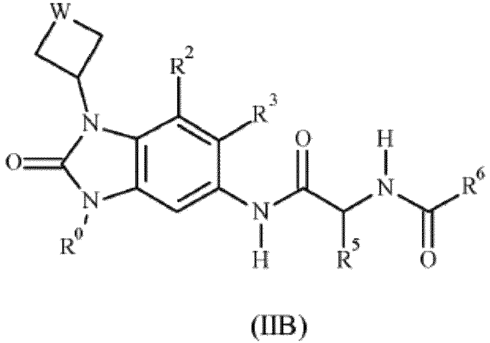
W représentant O ; et
R⁰, R², R³, R⁴, R⁵ et R⁶ étant tels que définis dans la revendication 1.

4. Composé selon la revendication 1 choisi parmi les suivants :
*N*-(1-cyclooctyl-2-{[1-(oxétan-3-yl)-2-oxo-3*H-*benzimidazol-5-yl]amino}-2-oxoéthyl)-3-méthylisoxazole-4-carboxamide ; et
*N*-(1-cyclooctyl-2-oxo-2-{[2-oxo-1-(tétrahydropyran-4-yl)-3*H*-benzimidazol-5-yl]-amino}éthyl)-3-méthylisoxazole-4-carboxamide.

5. Composé de formule (IA) tel que défini dans la revendication 1 ou *N*-oxyde correspondant ou sel pharmaceutiquement acceptable correspondant, pour une utilisation en thérapie.

6. Composé de formule (IA) tel que défini dans la revendication 1 ou *N*-oxyde correspondant ou sel pharmaceutiquement acceptable correspondant, pour une utilisation dans le traitement et/ou la prévention d'un trouble inflammatoire ou auto-immun .

7. Composition pharmaceutique comprenant un composé de formule (IA) tel que défini dans la revendication 1 ou un *N*-oxyde correspondant ou un sel pharmaceutiquement acceptable correspondant, en association avec un support pharmaceutiquement acceptable.

8. Composition pharmaceutique selon la revendication 7 comprenant en outre un ingrédient pharmaceutiquement actif supplémentaire.
